# EUROPEAN PATENT APPLICATION

(11) **EP 4 421 159 A1**
(43) Date of publication of application: **28.08.2024**
(21) Application number: 22900588.9
(22) Date of filing: 01.12.2022
(51) Int. Cl.: C12M 1/26, C12M 1/00

(54) **AUXILIARY CONNECTION DEVICE**

(30) Priority: 01.12.2021 CN 202111457291
(71) Applicant: Nanjing GenScript Biotech Co., Ltd., Nanjing, Jiangsu 211100 (CN)
(72) Inventor: ZHANG, Hao, Nanjing, Jiangsu 211100 (CN); WANG, Lin, Nanjing, Jiangsu 211100 (CN); SHI, Gaofeng, Nanjing, Jiangsu 211100 (CN); QIAN, Hong, Nanjing, Jiangsu 211100 (CN)
(74) Representative: Wang, Bo
(86) International application number: PCT/CN2022/135778
(87) International publication number: WO 2023/098784

(57) **Abstract**

Provided in an embodiment of the present description is an auxiliary connection device which comprises a power unit and a liquid transfer tubing assembly. The liquid transfer tubing assembly comprises a first liquid transfer tubing. The first liquid transfer tubing comprises a first port and a second port. The power unit engages with the first liquid transfer tubing, and is located at a first liquid transfer tube between the first port and the second port.

## Description

### Cross Reference to Related Application

This application is a U.S. national stage under 35 U.S.C. §371 of International Application No. PCT/CN2022/135778, filed on December 1, 2022, which claims priority to Chinese Patent Application No. 202111457291.3, filed on December 1, 2021, which is incorporated herein by reference in its entirety.

### Technical Field

This specification relates to the technical field of cell treatment devices, and in particular to an auxiliary connection device.

### Background Art

With the technological development in recent years, cell therapy has become a popular field of research and development in the biomedical industry. The cell therapy technological process is relatively complex. A variety of cell treatment devices are involved in the preparation of target cell products, and the cell treatment devices are independent of each other. When the staff follows the corresponding steps to transfer a cellular solution in the cell treatment devices, it is necessary to transfer the cellular solution to other cell treatment devices manually. This manual transfer method has the problem of a low efficiency. In addition, the frequent transfer of the cellular solution carried out by an operator increases the risk of contamination.

Therefore, the present application provides an auxiliary connection device capable of being used to realize a liquid transfer function between different cell treatment devices.

### Summary of the Invention

One of embodiments of this specification provides an auxiliary connection device, which includes: a power unit and a liquid transfer tubing assembly, where the liquid transfer tubing assembly includes a first liquid transfer tubing, the first liquid transfer tubing includes a first port and a second port, the power unit engages with the first liquid transfer tubing, and the power unit is located at the first liquid transfer tubing between the first port and the second port.

In some embodiments, the liquid transfer tubing assembly includes a second liquid transfer tubing, and the second liquid transfer tubing is connected to the temporary storage unit and the first liquid transfer tubing; a joint of the first liquid transfer tubing and the second liquid transfer tubing is located between the second port and the power unit; and the liquid transfer tubing assembly includes a third liquid transfer tubing, the third liquid transfer tubing is connected to the second liquid transfer tubing and the first liquid transfer tubing, and a joint of the third liquid transfer tubing and the first liquid transfer tubing is located between the first port and the power unit.

In some embodiments, the first liquid transfer tubing between the first port and the power unit is provided with a first valve, the first liquid transfer tubing between the second port and the power unit is provided with a second valve, the second liquid transfer tubing is provided with a third valve, and the third liquid transfer tubing is provided with a fourth valve.

In some embodiments, the device includes a casing, and the first liquid transfer tubing, the second liquid transfer tubing, and the third liquid transfer tubing are distributed on a side wall of the casing.

In some embodiments, the first valve, the second valve, the third valve and the fourth valve are pinch valves, and the pinch valves are connected to the side wall of the casing, wherein the pinch valves are located on an outer side of the liquid transfer tubing assembly.

In some embodiments, the power unit is a peristaltic pump, the peristaltic pump is located on the casing, and the first liquid transfer tubing engages with a pump head of the peristaltic pump.

In some embodiments, the device further includes a thermal insulation unit for providing a preset temperature, the thermal insulation unit is located in the casing, and the temporary storage unit is located in the thermal insulation unit.

In some embodiments, the device further includes a sampling unit, the liquid transfer tubing assembly includes a fourth liquid transfer tubing, the fourth liquid transfer tubing is connected to the sampling unit and the first liquid transfer tubing, and a joint of the fourth liquid transfer tubing and the first liquid transfer tubing is located between the second port and the power unit.

In some embodiments, the fourth liquid transfer tubing is provided with a fifth valve that controls the on/off state of the fourth liquid transfer tubing, and the fifth valve is a pinch valve, is connected to the casing, and is located on an outer side of the fourth liquid transfer tubing.

In some embodiments, the sampling unit is located in the thermal insulation unit.

In some embodiments, the device further includes a heating assembly, the heating assembly is located on the side wall of the casing, and the heating assembly is configured to cut off the fourth liquid transfer tubing and seal the cut end of the fourth liquid transfer tubing.

In some embodiments, the heating assembly is a heat sealing machine.

In some embodiments, the device includes a detection unit, the detection unit is located on an outer side of the first liquid transfer tubing between the first port and the power unit, and the detection unit is configured to determine whether there is a solution in the first liquid transfer tubing between the first port and the power unit.

In some embodiments, the detection unit is a bubble sensor, the bubble sensor is located on the outer side of the first liquid transfer tubing, and the bubble sensor determines, based on ultrasound, whether there is a solution in the first liquid transfer tubing between the first port and the power unit.

In some embodiments, the device further includes an information prompting unit connected to the detection unit, and the information prompting unit sends a prompt message in response to a feedback from the detection unit.

In some embodiments, the device further includes a rinsing unit, the liquid transfer tubing assembly includes a fifth liquid transfer tubing, one end of the fifth liquid transfer tubing is connected to the rinsing unit, and the other end of the fifth liquid transfer tubing is connected to the third liquid transfer tubing or the second liquid transfer tubing.

In some embodiments, the fifth liquid transfer tubing is provided with a sixth valve.

In some embodiments, the second liquid transfer tubing includes a first sub-tubing and a second sub-tubing connected in sequence, a port of the first sub-tubing is connected to the temporary storage unit, a port of the second sub-tubing is connected to the first liquid transfer tubing, the third valve is located on the first sub-tubing, and the second sub-tubing is provided with a seventh valve.

In some embodiments, the device further includes a supporting member, and the rinsing unit is disposed on the supporting member.

### Brief Description of the Drawings

This specification will be further illustrated by way of exemplary embodiments, and these exemplary embodiments will be described in detail with reference to the accompanying drawings. These embodiments are not restrictive, and in these embodiments, the same numerals represent the same structures, in which:
FIG. 1 is a tubing diagram of an auxiliary connection device according to some embodiments in this specification;
FIG. 2 is a tubing diagram of another auxiliary connection device according to some embodiments in this specification;
FIG. 3 is a schematic structural diagram of an auxiliary connection device according to some embodiments in this specification;
FIG. 4 is another schematic structural diagram of an auxiliary connection device according to some embodiments in this specification;
FIG. 5 is a schematic structural diagram of a cell culture system according to some embodiments in this specification; and
FIG. 6 is a schematic structural diagram of another cell culture system according to some embodiments in this specification.

### Detailed Description of Embodiments

For a clearer description of the technical solutions in the embodiments of this specification, the accompanying drawings required for describing the embodiments will be briefly described below. Apparently, the accompanying drawings in the following description show merely some examples or embodiments of this specification, and those of ordinary skill in the art may still apply this specification to other similar scenarios according to these accompanying drawings without any creative effort. Unless obvious from the linguistic context or otherwise stated, the same reference signs in the drawings represent the same structure or operation.

It should be understood that "system", "device", "unit" and/or "module" as used herein is a method for distinguishing different components, elements, parts, portions or assemblies of different levels. However, these words may be substituted by other expressions if these expressions accomplish the same purpose.

As shown in this specification and the claims, the words "one", "a", "an" and/or "the" do not specifically refer to the singular, but may also include the plural, unless the context clearly indicates otherwise. Generally, the terms "including" and "comprising" only imply the inclusion of explicitly identified steps and elements, and these steps or elements do not constitute an exclusive list. A method or a device may further comprise other steps or elements.

Flowcharts are used in this specification to illustrate operations performed by a system according to the embodiments of this specification. It should be understood that preceding or following operations are not necessarily performed in an exact order. Instead, all steps may be executed in a reverse order or simultaneously. Moreover, it is possible to add other operations to these processes, or remove a step or several steps from these processes.

In the technological process of cell therapy, a variety of cell treatment devices are involved in the preparation of target cell products. Because the cell treatment devices are independent of each other, corresponding parameters of a cellular solution need to be detected after the cellular solution is treated via the cell treatment devices, and then, the cellular solution is allowed to enter a next cell treatment device. In this process, in order to ensure physiological activity of the cellular solution, the cellular solution needs to be collected and stored manually in the process of testing the corresponding parameters. The method of manual operation is not only low in efficiency, but also has a high risk of contamination after the cellular solution is transferred multiple times, resulting in the obtained cell product failing to meet the expected requirements.

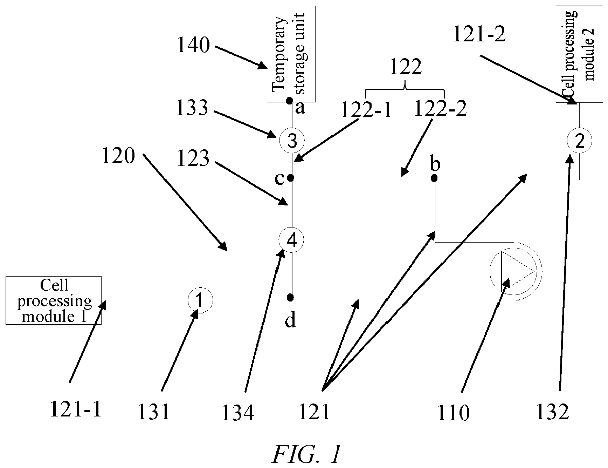

This specification mainly describes an auxiliary connection device. The auxiliary connection device is mainly configured to connect cell processing modules (e.g., a cell processing module 1 and a cell processing module 2 shown in FIG. 1) involved in cell treatment processes. The auxiliary connection device can transfer a solution from the cell processing module 1 to the cell processing module 2, which replaces the method of manual transfer. It should be noted that the auxiliary connection device herein is an independent device, and the auxiliary connection device can be detachably connected to the cell processing modules; the cell processing module 1 and the cell processing module 2 in the specification are only for better illustrating the function and effect of the auxiliary connection device; and the auxiliary connection device in this specification can flexibly combine and install the various cell processing modules according to different cell treatment processes.

In some embodiments, the auxiliary connection device mainly includes a power unit and a liquid transfer tubing assembly engaging with the power unit. The power unit is mainly configured to provide a power source to drive a solution in the liquid transfer tubing assembly to flow. In some embodiments, the liquid transfer tubing assembly may include a first liquid transfer tubing, the first liquid transfer tubing includes a first port and a second port, the first liquid transfer tubing engages with the power unit, the power unit is located at a tubing between the first port and the second port, and the first port and the second port of the first liquid transfer tubing are connected to cell processing modules involved in the cell treatment processes, respectively. The first liquid transfer tubing transfers the solution under the action of the power unit while the airtightness of a solution transfer process is ensured to isolate the solution from the external world, thereby preventing the solution from being contaminated during the transfer process, and improving the safety of the solution transfer process.

In some embodiments, the auxiliary connection device may further include a temporary storage unit, and the liquid transfer tubing assembly may further include a second liquid transfer tubing. The second liquid transfer tubing is connected to the temporary storage unit and the first liquid transfer tubing. The joint of the first liquid transfer tubing and the second liquid transfer tubing is located between the second port and the power unit. The temporary storage unit can store the solution to be transferred, thereby facilitating subsequent operations of the solution to be transferred. In some embodiments, the liquid transfer tubing assembly may further include a third liquid transfer tubing, and the third liquid transfer tubing is connected to the second liquid transfer tubing and the first liquid transfer tubing. The joint of the third liquid transfer tubing and the first liquid transfer tubing is located between the first port and the power unit, and the solution to be transferred from the temporary storage unit enters the cell processing module 2 through the second liquid transfer tubing, the third liquid transfer tubing and the first liquid transfer tubing. During operation of the auxiliary connection device, under the drive of the power unit, the solution from the cell processing module 1 enters the temporary storage unit for storage through the first liquid transfer tubing and the second liquid transfer tubing. After the solution is sampled and tested and meets conditions, the power unit acts on the liquid transfer tubing assembly, and the solution in the temporary storage unit sequentially passes through a portion of the second liquid transfer tubing (i.e., the second liquid transfer tubing between the temporary storage unit and the third liquid transfer tubing) and the third liquid transfer tubing to enter the first liquid transfer tubing, and finally to enter the cell processing module 2. In some embodiments, the cell processing module 1 and the cell processing module 2 may be the same cell processing modules or different cell processing modules, which can be adaptively adjusted according to the corresponding cell treatment processes.

The auxiliary connection device provided in the embodiments of this specification realizes the storage and transfer of the solution through the first liquid transfer tubing, the second liquid transfer tubing, and the third liquid transfer tubing of the liquid transfer tubing assembly. In the process of transferring the solution, the auxiliary connection device can provide a closed and integrated confined space for the solution, reducing the contact between the solution and an external environment, decreasing the risk of the solution being contaminated, and accordingly increasing the success rate of cell experiments or products. In addition, with the auxiliary connection device, workload of the staff is reduced while efficiency of the cell experiments or production is improved.

FIG. 1 is a tubing diagram of an auxiliary connection device according to some embodiments in this specification. As shown in FIG. 1, the auxiliary connection device may include a power unit 110, a liquid transfer tubing assembly 120 engaging with the power unit 110, and a temporary storage unit 140 connected to the liquid transfer tubing assembly 120.

The power unit 110 is mainly configured to provide a power source to drive the solution to flow, so that the solution is allowed to flow within the liquid transfer tubing assembly 120. In some embodiments, the power unit 110 may be a peristaltic pump, and the liquid transfer tubing assembly 120 engages with a pump head of the peristaltic pump. In some embodiments, a part of tubing of the liquid transfer tubing assembly 120 that engages with the pump head of the peristaltic pump is a hose, and the hose is placed between the pump head of the peristaltic pump and a shell. When the pump head of the peristaltic pump is rotated, a plurality of squeezing members on the pump head alternately squeeze and release the hose, and the hose is squeezed by the squeezing member to form a closing point. When the pump head is rotated, the closing point follows the rotation of the pump head, and at the same time, the solution in front of the closing point also moves with the pump head. The hose will return to its natural state after the squeezing member leaves, and a vacuum will be formed inside the hose, so that the solution is aspirated and squeezed out by the next squeezing member. Moreover, because the hose is squeezed by the squeezing member to be closed completely, the solution will not flow back. The peristaltic pump can precisely pump the solution in a small volume, and does not come into contact with the solution when the solution is pumped, the solution only comes into contact with an outer side of the liquid transfer tubing assembly 120, thus achieving the effect of aseptic and clean transfer. It should be noted that the power unit 110 is not limited to the peristaltic pump described above, but may also be other power devices.

In some embodiments, the liquid transfer tubing assembly 120 may include one or more of a rigid tube, a flexible tube, and the like. Preferably, for easier and more flexible installation, the liquid transfer tubing assembly 120 may utilize flexible tubes. In some embodiments, the liquid transfer tubing assembly 120 may use transparent tubes in order to bring convenience to an operator to observe the transfer of the solution.

With continued reference to FIG. 1, in some embodiments, the liquid transfer tubing assembly 120 may include a first liquid transfer tubing 121. The first liquid transfer tubing 121 may include a first port 121-1 and a second port 121-2. The first port 121-1 may be connected to the cell processing module 1, and the second port 121-2 may be connected to the cell processing module 2, thereby realizing transfer of the solution between the cell processing module 1 and the cell processing module 2. It should be noted that the cell processing module 1 and the cell processing module 2 are proposed for the purpose of conveniently describing the function and effect of the auxiliary connection device, and are not structural components of the auxiliary connection device. Herein, the power unit 110 engages with the first liquid transfer tubing 121 so as to provide power for transferring the solution from the cell processing module 1 to the cell processing module 2.

The temporary storage unit 140 is mainly configured to temporarily store the solution that has been treated by the cell processing module 1, thereby facilitating subsequent operations by the operator, such as preserving heat of the solution. In some embodiments, the temporary storage unit 140 may include one or more of a solution bag, a solution box, and the like, as long as it has good airtightness and does not react with the stored solution. This specification does not impose excessive limitations thereon.

In some embodiments, the liquid transfer tubing assembly 120 may also include a second liquid transfer tubing 122 (segments a-c-b illustrated in FIG. 1) for providing a channel for the solution to enter the temporary storage unit 140, and the second liquid transfer tubing 122 is connected to the temporary storage unit 140 and the first liquid transfer tubing 121. Specifically, one end of the second liquid transfer tubing 122 is connected to the temporary storage unit 140, and the other end of the second liquid transfer tubing 122 is connected to the first liquid transfer tubing 121. The joint (at point b illustrated in FIG. 1) of the first liquid transfer tubing 121 and the second liquid transfer tubing 122 is located between the second port 121-2 and the power unit 110. Since the solution needs to be pumped by the power unit 110 such that there is power for driving the solution to flow within the liquid transfer tubing assembly 120, the joint b of the first liquid transfer tubing 121 and the second liquid transfer tubing 122 is located between the second port 121-2 and the power unit 110, so that the solution enters the first liquid transfer tubing 121 from the first port 121-1 and then passes through the second liquid transfer tubing 122 to finally enter the temporary storage unit 140 for storage.

In some embodiments, the liquid transfer tubing assembly 120 may further include a third liquid transfer tubing 123 (segment c-d illustrated in FIG. 1) for providing a channel for discharging the solution out of the temporary storage unit 140, and the third liquid transfer tubing 123 is connected to the second liquid transfer tubing 122 and the first liquid transfer tubing 121. The joint (point d illustrated in FIG. 1) of the third liquid transfer tubing 123 and the first liquid transfer tubing 121 is located between the first port 121-1 and the power unit 110. Since the solution needs to be pumped by the power unit 110 such that there is power for driving the solution to flow within the liquid transfer tubing assembly 120, the joint d of the third liquid transfer tubing 123 and the first liquid transfer tubing 121 is located between the first port 121-1 and the power unit 110. Under the action of the power unit 110, the solution in the temporary storage unit 140 passes through a portion of the second liquid transfer tubing 122 (i.e., the second liquid transfer tubing 122 between the temporary storage unit 140 and the third liquid transfer tubing 123) and the third liquid transfer tubing 123 to enter the first liquid transfer tubing 121, and then enters the cell processing module 2.

To facilitate the control on a flow direction of the solution, in some embodiments, a corresponding liquid transfer tubing of the liquid transfer tubing assembly 120 may be provided with a valve. In some embodiments, the first liquid transfer tubing 121 between the first port 121-1 and the power unit 110 is provided with a first valve 131, and the first valve 131 is configured to control connection or disconnection of the first liquid transfer tubing 121 between the first port 121-1 and the power unit 110. The first liquid transfer tubing 121 between the second port 121-2 and the power unit 110 is provided with a second valve 132, and the second valve 132 is configured to control connection or disconnection of the first liquid transfer tubing 121 between the second port 121-2 and the power unit 110. The second liquid transfer tubing 122 is provided with a third valve 133, and the third valve 133 is configured to control connection or disconnection of the second liquid transfer tubing 122 between the first liquid transfer tubing 121 and the temporary storage unit 140. The third liquid transfer tubing 123 is provided with a fourth valve 134, and the fourth valve 134 is configured to control connection or disconnection of the third liquid transfer tubing 123 between the first liquid transfer tubing 121 and the second liquid transfer tubing 122.

In some embodiments, the first valve 131, the second valve 132, the third valve 133, and the fourth valve 134 may all be pinch valves, and the liquid transfer tubing assembly 120 may be a flexible tube. Connection or disconnection of the corresponding tubings of the liquid transfer tubing assembly may be controlled by controlling the degree of squeezing of the pinch valves on the liquid transfer tubing assembly 120. The pinch valves are located on the outer side of the liquid transfer tubing assembly 120, and can secure the corresponding liquid transfer tubings. Moreover, the pinch valves can also avoid contact between the valves and the solution in the tubing, ensuring that the solution is not in contact with the external environment during the solution transfer process, and reducing the possibility of contamination during the solution transfer process. On the other hand, since the pinch valves mainly control the connection or disconnection of each tubing in the corresponding liquid transfer tubing by controlling the degree of squeezing to the liquid transfer tubing assembly 120, the pinch valves are separable from the corresponding liquid transfer tubings, and the pinch valves may not need to be replaced in the subsequent process of replacing the liquid transfer tubing assembly 120 of the auxiliary connection device. It should be noted that the first valve 131, the second valve 132, the third valve 133 and the fourth valve 134 are not limited to the pinch valves described above, but may also be other types of valves, for example, a flow regulating switch on a medical infusion tube. In addition, when the auxiliary connection device is applied to other scenarios (e.g., scenarios in which the requirements for the transfer of the solution are not high), the types of the first valve 131, the second valve 132, the third valve 133, and the fourth valve 134 may also be corresponding types of valves selected according to the type of the liquid transfer tubing assembly 120. Illustratively, when the liquid transfer tubing assembly 120 is a rigid tubing, the valves may be selected from plug valves.

In order to illustrate the liquid transfer tubing assembly 120 in the auxiliary connection device more clearly, the following description is given in conjunction with different transfer situations of the solution. When the solution is transferred directly from the cell processing module 1 to the cell processing module 2, the first valve 131 and the second valve 132 are opened and the third valve 133 and the fourth valve 134 are closed. Under the action of the power unit 110, the solution is discharged from the cell processing module 1, enters the first liquid transfer tubing 121 through the first port 121-1, and then is discharged from the second port 121-2 to enter the cell processing module 2.

When the solution is transferred from the cell processing module 1 to the temporary storage unit 140, the first valve 131 and the third valve 133 are opened and the second valve 132 and the fourth valve 134 are closed. Under the action of the power unit 110, the solution is discharged from the cell processing module 1, enters the first liquid transfer tubing 121 through the first port 121-1, and enters the second liquid transfer tubing 122 through a portion of the first liquid transfer tubing 121 (i.e., the first liquid transfer tubing 121 between the cell processing module 1 and the second liquid transfer tubing 122) to enter the temporary storage unit 140.

When the solution is transferred from the temporary storage unit 140 to the cell processing module 2, the second valve 132, the third valve 133 and the fourth valve 134 are opened and the first valve 131 is closed. Under the action of the power unit 110, the solution is discharged from the temporary storage unit 140, enters the first liquid transfer tubing 121 through a portion of the second liquid transfer tubing 122 (i.e., the second liquid transfer tubing 122 between the temporary storage unit 140 and the third liquid transfer tubing 123), and the third liquid transfer tubing 123, to enter the cell processing module 2.

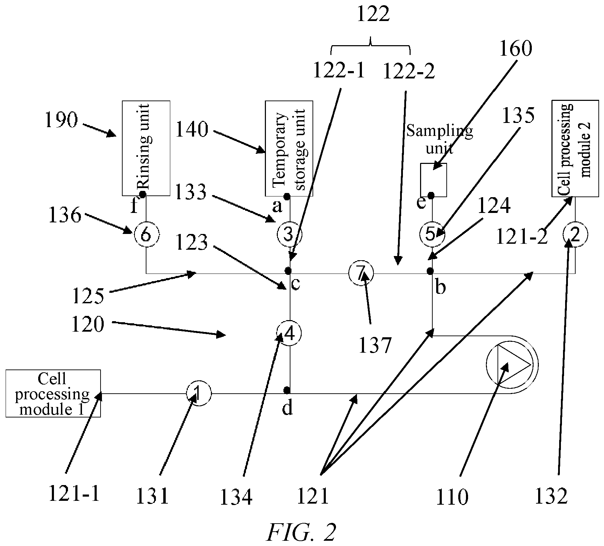

FIG. 2 is a tubing diagram of another auxiliary connection device according to some embodiments in this specification.

As shown in FIG. 2, in order to facilitate detection of the solution treated previously (e.g., the solution treated by the cell processing module 1), in some embodiments, the auxiliary connection device may further include a sampling unit 160, and the liquid transfer tubing assembly 120 may further include a fourth liquid transfer tubing 124 (segment e-b illustrated in FIG. 2). The sampling unit 160 is connected to the first liquid transfer tubing 121 via the fourth liquid transfer tubing 124. Under the action of the power unit 110, the solution in the solution cell processing module 1 enters the fourth liquid transfer tubing 124 through a portion of the first liquid transfer tubing 121 (i.e., the first liquid transfer tubing 121 between the cell processing module 1 and the fourth liquid transfer tubing 124), and flows to the sampling unit 160. The operator may sample the solution from the sampling unit 160 for subsequent operations, such as testing and analyzing and sample preservation.

In some embodiments, the sampling unit 160 may be removable with respect to the fourth liquid transfer tubing 124, which brings convenience to the operator to directly remove the sampling unit 160 for the subsequent operations. The removable connection here may refer to a snap fit, a bonding, a threaded connection, and the like. In some embodiments, the sampling unit 160 may be fixedly disposed, and accordingly the sampling unit 160 is provided with a sampling hole that may be opened and closed. The operator may extract the solution within the sampling unit 160 from the sampling hole for the subsequent operations. In some embodiments, the sampling unit 160 may include, but is not limited to, a container such as a solution bag, a solution box, a test tube, a storage tank, and the like.

In some embodiments, since the solution needs to be pumped by the power unit 110 such that there is power for driving the solution to flow within the liquid transfer tubing assembly 120, the joint of the fourth liquid transfer tubing 124 and the first liquid transfer tubing 121 is located between the second port 121-2 and the power unit 110. After the solution enters the first liquid transfer tubing 121 under the action of the power unit 110, it enters the fourth liquid transfer tubing 124 through a portion of the first liquid transfer tubing 121 (i.e., the first liquid transfer tubing 121 between the cell processing module 1 and the fourth liquid transfer tubing 124), and finally enters the sampling unit 160.

In some embodiments, the fourth liquid transfer tubing 124 is provided with a fifth valve 135, and the fifth valve 135 is configured to control the on/off state of the fourth liquid transfer tubing 124. In some embodiments, the fifth valve 135 may be a pinch valve, and the fifth valve 135 is located on an outer side of the fourth liquid transfer tubing 124. The specific condition of the fifth valve 135 may be referred to the related description of the valves such as the first valve 131 above and will not be repeated herein.

When part of the solution is transferred from the cell processing module 1 to the sampling unit 160, the first valve 131 and the fifth valve 135 are opened, and the second valve 132, the third valve 133 and the fourth valve 134 are closed. The solution is discharged from the cell processing module 1, enters the first liquid transfer tubing 121 from the first port 121-1, is pumped by the power unit 110 through the first valve 131, then flows through the first liquid transfer tubing 121 to enter the fourth liquid transfer tubing 124, and passes through the fifth valve 135 to enter the sampling unit 160 from the fourth liquid transfer tubing 124.

Since the sampling unit 160 is mainly configured to sample the solution for the subsequent operations, the sampling unit 160 has a relatively small capacity, occupies a small portion of the solution as a whole, and has less effect on the transfer of the solution. Therefore, in some embodiments, when the solution is transferred out of the cell processing module 1, the solution can be transferred to the sampling unit 160 and the temporary storage unit 140 at the same time. At this time, the first valve 131, the third valve 133 and the fifth valve 135 are opened, and the second valve 132 and the fourth valve 134 are closed. The solution is discharged from the cell processing module 1, enters the first liquid transfer tubing 121 from the first port 121-1, is pumped by the power unit 110 through the first valve 131, flows through the first liquid transfer tubing 121, and enters the second liquid transfer tubing 122 and the fourth liquid transfer tubing 124 at the same time. The solution entering the second liquid transfer tubing 122 flows through the third valve 133 and then enters the temporary storage unit 140. The solution entering the fourth liquid transfer tubing 124 flows through the fifth valve 135 and then enters the sampling unit 160.

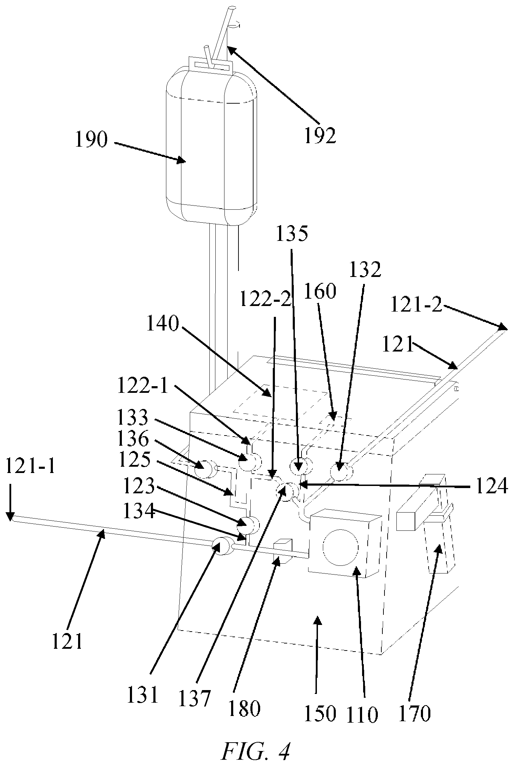

When the auxiliary connection device collects the solution through the temporary storage unit 140 or the sampling unit 160, there may be residual solution in the corresponding tubing of the liquid transfer tubing assembly 120. It may take some time for the detection of the solution. If the time is long, the component property (e.g., cells) of the residual solution in the tubing may change. In order to prevent the residual solution in the liquid transfer tubing assembly 120 from affecting the subsequent treatment, as shown in FIG. 4, in some embodiments, the auxiliary connection device 100 may further include a rinsing unit 190. The rinsing unit 190 may be a container containing a rinsing liquid (e.g., a phosphate buffer solution), such as a solution bag and a solution bottle, etc. In some embodiments, the liquid transfer tubing assembly 120 may further include a fifth liquid transfer tubing 125 (segment f-c illustrated in FIG. 2). One end of the fifth liquid transfer tubing 125 is connected to the rinsing unit 190, and the other end of the fifth liquid transfer tubing 125 is connected to the third liquid transfer tubing 123 or the second liquid transfer tubing 122. The rinsing liquid in the rinsing unit 190 may enter the liquid transfer tubing assembly 120 through the fifth liquid transfer tubing 125 and rinse the liquid transfer tubing assembly 120.

In some embodiments, the fifth liquid transfer tubing 125 is provided with a sixth valve 136. The sixth valve 136 is capable of controlling connection or disconnection of the fifth liquid transfer tubing 125, thereby controlling whether the rinsing unit 190 works or not.

In some embodiments, the second liquid transfer tubing 122 may include a first sub-tubing 122-1 (segment a-c illustrated in FIG. 2) and a second sub-tubing 122-2 (segment c-b illustrated in FIG. 2) sequentially connected. A port of the first sub-tubing 122-1 is connected to the temporary storage unit 140, a port of the second sub-tubing 122-2 is connected to the first liquid transfer tubing 121, the third valve 133 is located on the first sub-tubing 122-1, and the second sub-tubing 122-2 is provided with a seventh valve 137. The seventh valve 137 is capable of controlling connection or disconnection between the second liquid transfer tubing 122 and the first liquid transfer tubing 121, and also controlling connection or disconnection between the fifth liquid transfer tubing 125 and the first liquid transfer tubing 121 at the same time.

In some embodiments, when the rinsing unit 190 works, the first valve 131, the sixth valve 136 and the seventh valve 137 are opened, and the second valve 132, the third valve 133, the fourth valve 134 and the fifth valve 135 are closed. At this time, under the action of the power unit 110, the rinsing liquid in the rinsing unit 190 enters the second sub-tubing 122-1 through the sixth valve 136, enters the first liquid transfer tubing 121 through the seventh valve 137, and is then pumped by the power unit 110 to flow out of the first port 121-1 of the first liquid transfer tubing 121.

In some embodiments, when the rinsing unit 190 works, the second valve 132, the fourth valve 134 and the sixth valve 136 are opened, and the first valve 131, the third valve 133, the fifth valve 135 and the seventh valve 137 are closed. At this time, under the action of the power unit 110, the rinsing liquid in the rinsing unit 190 enters the third liquid transfer tubing 123 through the sixth valve 136, enters the first liquid transfer tubing 121 through the fourth valve 134, and flows out of the second port 121-2 of the first liquid transfer tubing 121.

It should be noted that the seventh valve 137 is not limited to being opened or closed when the rinsing unit 190 works; and when the cellular solution needs to be transferred to other modules (the cell processing module 2) or other units (e.g., the temporary storage unit 140 and the sampling unit 160), by controlling an open or close state of the seventh valve 137, a transfer path of the cellular solution can be better controlled. For example, when part of the cellular solution in the cell processing module 1 is transferred to the sampling unit 160, the first valve 131 and the fifth valve 135 are opened, and the second valve 132, the third valve 133, the fourth valve 134 and the seventh valve 137 are closed. As another example, when the solution is transferred out of the cell processing module 1, it may be transferred to the sampling unit 160 and the temporary storage unit 140 at the same time. At this time, the first valve 131, the third valve 133, the fifth valve 135 and the seventh valve 137 are opened, and the second valve 132 and the fourth valve 134 are closed. As another example, when the solution in the cell processing module 1 is transferred to the cell processing module 2, the first valve 131 and the second valve 132 are opened, and the third valve 133, the fourth valve 134, the fifth valve 135, the sixth valve 136 and the seventh valve 137 are closed.

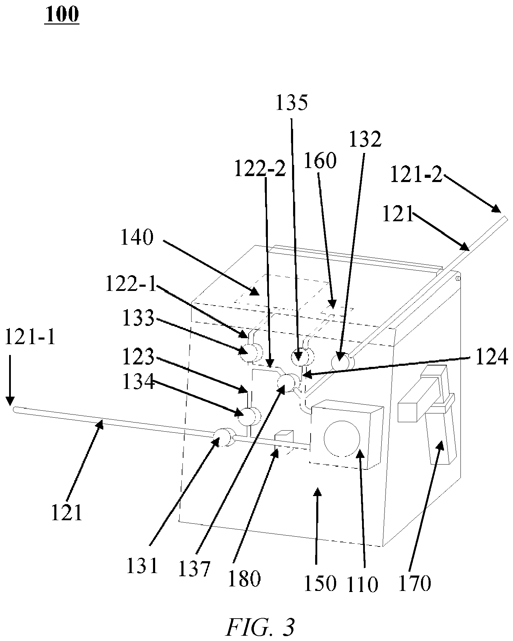

FIG. 3 is a schematic structural diagram of an auxiliary connection device according to some embodiments in this specification. As shown in FIG. 3, in some embodiments, the auxiliary connection device 100 may further include a casing 150. The casing 150 mainly serves as a supporting body and provides a mounting platform for other components (e.g., the liquid transfer tubing assembly 120, the valves, the power unit 110, the temporary storage unit 140, and the sampling unit 160) of the auxiliary connection device 100. In some embodiments, the casing 150 may be of a cubic structure, the interior of the casing 150 has a holding space, and the power unit 110, the temporary storage unit 140, and the sampling unit 160 may be disposed in the holding space of the casing 150. In some embodiments, the first liquid transfer tubing 121, the second liquid transfer tubing 122, the third liquid transfer tubing 123, the fourth liquid transfer tubing 124, and the like are distributed on an outer side wall of the casing 150. In some embodiments, the pinch valves may be connected to the side wall of the casing 150. The pinch valves are located on the outer side of the liquid transfer tubing assembly 120 and have an effect of securing and limiting the liquid transfer tubing assembly 120.

Since the cell treatment process has a high requirement for the cleanliness of the cellular solution in order to ensure a desired result, it is necessary to avoid contamination of the cellular solution as much as possible. Therefore, after use of the auxiliary connection device 100, the liquid transfer tubing assembly 120 needs to be cleaned or replaced in order to prevent the residual solution in the liquid transfer tubing assembly 120 from contaminating a solution to be transferred the next time. In some embodiments, the liquid transfer tubing assembly 120 is mounted on the side wall of the casing 150, so that removal, installation, and replacement of the liquid transfer tubing assembly 120 are facilitated. It should be noted that when the liquid transfer tubing assembly 120 needs to be replaced, the pinch valves may not be replaced because the pinch valves are not in contact with the solution in the liquid transfer tubing assembly 120. Moreover, the liquid transfer tubing assembly 120 is mounted on the side wall of the casing 150, which brings convenience for the operator to observe the transfer of the solution in the liquid transfer tubing assembly 120, thus enabling the operator to have a clear understanding of the transfer of the solution in real time.

In some embodiments, the auxiliary connection device 100 may further include a thermal insulation unit (not shown in the figures). The thermal insulation unit is located in the holding space inside the casing 150. The holding space may be a confined space. The holding space of the casing 150 may provide a confined thermal insulation environment for the thermal insulation unit, and can isolate the thermal insulation unit from the outside world, and improve the thermal insulation performance of the thermal insulation unit. In some embodiments, the casing 150 and the side wall inside the holding space thereof may be provided with a thermal insulation material layer to improve the thermal insulation effect of the thermal insulation unit. In some embodiments, the material of the thermal insulation material layer may include, but is not limited to, one or more of polyurethane foam, polystyrene boards, phenolic foam, ceramic fiber blankets, aluminum silicate felt, alumina, silicon carbide fibers, aerogel felt, glass wool, rock wool, expanded perlite, micro-nano thermal insulation boards, foamed cement, and the like. In some embodiments, the thermal insulation unit may be a refrigerating type insulation device or a heating type insulation (e.g., resistance heating) device. In some embodiments, the thermal insulation unit may also utilize, but is not limited to, lowtemperature holding methods such as ice pack cooling and dry ice cooling. In some embodiments, the temperature of the thermal insulation unit may be set according to the specific solution insulation environment.

In some embodiments, the temporary storage unit 140 is located in the thermal insulation unit, and the thermal insulation unit can provide an external environment with a preset temperature for the solution stored in the temporary storage unit 140, so that the property of the solution remains stable. In some embodiments, when the solution within the temporary storage unit 140 is a cellular solution, the preset temperature of the thermal insulation unit may be 2-8°C in order to ensure physiological activity of cells. When the auxiliary connection device 100 is applied in other scenarios, for example, when the auxiliary connection device 100 is configured to store and transfer a conventional solution, the preset temperature of the thermal insulation unit may be a room temperature (e.g., about 25°C).

In some embodiments, the sampling unit 160 may be located in the thermal insulation unit. The thermal insulation unit can provide an external environment with a preset temperature for the solution stored in the sampling unit 160 (e.g., in the case that the solution within the temporary storage unit 140 is a cellular solution, the preset temperature of the thermal insulation unit may be 2-8°C), so that the property of the solution remains stable.

In some embodiments, the auxiliary connection device 100 may further include a heating assembly 170, and the heating assembly 170 may be disposed on the side wall of the casing 150. The heating assembly 170 is mainly configured to cut off the fourth liquid transfer tubing 124 and seal the cut end of the fourth liquid transfer tubing 124, thus avoiding the connection between the outside world and the fourth liquid transfer tubing 124, reducing the effect of the outside world on the solution in the fourth liquid transfer tubing 124, and reducing the possibility of the solution being contaminated.

In some embodiments, the heating assembly 170 may be a heat sealing machine, and the heat sealing machine is removably secured to the side wall of the casing 150 by a structure such as a hook. During use, the operator may directly remove the heat sealing machine and use the heat sealing machine to heat and fuse the fourth liquid transfer tubing 124 and seal the cut of the fourth liquid transfer tubing 124. The operator may directly remove the sampling unit 160 for the subsequent operations. In some embodiments, the heating assembly 170 may also utilize a structure such as an electrical iron.

In some embodiments, the auxiliary connection device 100 may further include a detection unit 180, and the detection unit 180 is mounted on an outer side of the first liquid transfer tubing 121 between the first port 121-1 and the power unit 110. The detection unit 180 is capable of determining whether there is a solution in the first liquid transfer tubing 121 between the first port 121-1 and the power unit 110. When the detection unit 180 determines that there is no solution in the first liquid transfer tubing 121 between the first port 121-1 and the power unit 110, it proves that the solution has been completely discharged, and there is no residual solution at a position where the solution is discharged (e.g., the cell processing module 1), and at this time, the power unit 110 can be turned off, and the corresponding valves can be closed.

In some embodiments, the detection unit 180 may be a bubble sensor, and the bubble sensor is located on the outer side of the first liquid transfer tubing 121. The bubble sensor is capable of detecting the presence of air bubbles within the first liquid transfer tubing 121. If air bubbles exist, it indicates that the solution in the first liquid transfer tubing 121 is mixed with the air bubbles or even that there is no more solution in the first liquid transfer tubing 121, and that there is insufficient solution remaining at the position where the solution is discharged or that the solution has been completely discharged, at this time, the power unit 110 can be turned off and the corresponding valves can be closed. In some embodiments, the detection unit 180 may further include, but is not limited to, an ultrasonic fluid sensor and the like. For example, the detection unit 180 may also be an infrared sensor. The infrared sensor includes a generator for emitting infrared rays, and a receiver. The generator and the receiver may be located on two sides of the first liquid transfer tubing 121 respectively, and are disposed opposite to each other, such that the receiver may receive infrared rays passing through the first liquid transfer tubing 121. The receiver outputs an electrical signal after receiving the infrared rays passing through the first liquid transfer tubing 121. A processing unit of the infrared sensor determines whether there are air bubbles in the first liquid transfer tubing 121 on the basis of the electrical signal. It should be noted that when the detection unit 180 is an infrared sensor, the infrared sensor may be located in a closed environment to prevent the external environment from affecting its detection.

In some embodiments, the detection unit 180 may be located to the right of the joint between the third liquid transfer tubing 123 and the first liquid transfer tubing 121, that is, the detection unit 180 is located between the joint and the power unit 110. Here, the detection unit 180 is capable of simultaneously determining whether the solution in the cell processing module 1 or the temporary storage unit 140 is completely discharged. In some embodiments, the detection unit 180 may be located to the left of the joint between the third liquid transfer tubing 123 and the first liquid transfer tubing 121, that is, the detection unit 180 is located between the joint and the first port 121-1. Here, the detection unit 180 is only capable of detecting whether the solution within the cell processing module 1 is completely discharged. In some embodiments, the auxiliary connection device 100 may further include an information prompting unit (not shown in the figures) connected to the detection unit 180, and the information prompting unit can send a prompt message in response to a feedback from the detection unit 180.

In some embodiments, the information prompting unit may include an alarm light (not shown in the figures) disposed on an outer wall of the casing 150. In some embodiments, the information prompting unit may include, but is not limited to, an audible prompter, such as an alarm bell. In some embodiments, the information prompting unit may further include, but is not limited to, a text prompter such as a display. In the case that there is no solution in the first liquid transfer tubing 121, the information prompting unit gives a corresponding prompt that the solution has been completely transferred, thus reminding the operator to turn off the power unit 110.

In some embodiments, the auxiliary connection device 100 may further include a supporting member 192. The rinsing unit 190 may be disposed on the supporting member 192, and the supporting member 192 secures the rinsing unit 190. In some embodiments, the supporting member 192 may be a retractable structure, bringing convenience for the operator to lift and lower the rinsing unit 190 so as to operate the rinsing unit 190.

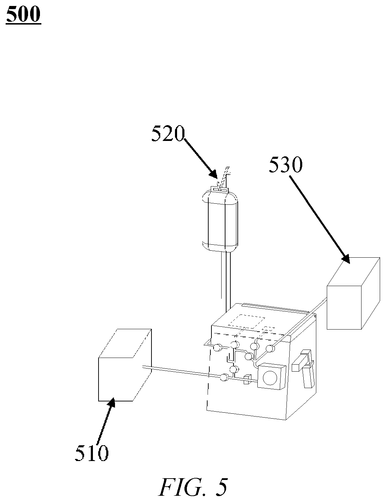

This specification further provides a cellular solution treatment system. FIG. 5 is a schematic structural diagram of a cell treatment system 500 according to an embodiment in this specification. As shown in FIG. 5, the cell treatment system 500 may include a cell treatment device 510, a cell treatment device 530, and an auxiliary connection device 520 for connecting the two. Specific contents regarding the auxiliary connection device 520 may be referred to the related description of the auxiliary connection device 100 described above. In some embodiments, the cell treatment device 510 and the cell treatment device 530 may be the same cell treatment device or may be different cell treatment devices. For example, both the cell treatment device 510 and the cell treatment device 530 may be one of a cell washing device, a cell incubation device, or a cell sorting device. As another example, the cell treatment device 510 may be a cell washing device and the cell treatment device 530 may be a cell incubation device. As another example, the cell treatment device 510 may be a cell sorting device and the cell treatment device 530 may be a cell culture device. It should be noted that the above description of the cell treatment device 510 and the cell treatment device 530 is only for exemplary illustration, and the cell treatment device 510 and the cell treatment device 530 can be adaptively adjusted according to actual application scenarios or cell treatment processes.

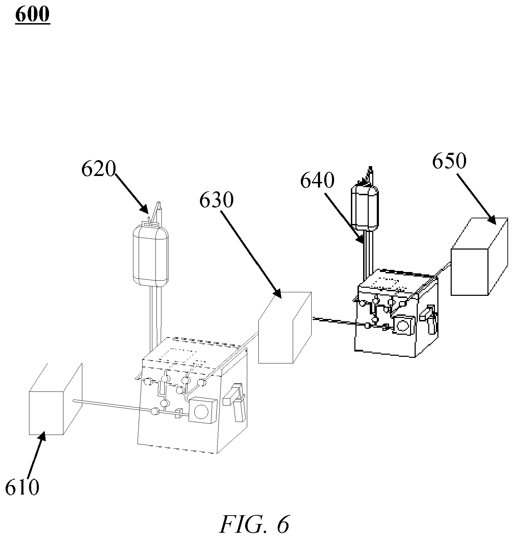

FIG. 6 is a schematic structural diagram of a cell treatment system 600 according to an embodiment in this specification. As shown in FIG. 6, the cell treatment system 600 may include a first cell treatment device 610, a second cell treatment device 630, and a third cell treatment device 650. The first cell treatment device 610 is connected to the second cell treatment device 630 through a first auxiliary connection device 620, and the second cell treatment device 630 is connected to the third cell treatment device 650 through a second auxiliary connection device 640. It should be noted that the cell treatment system 600 is only an example of the auxiliary connection device in applications, and the position of the auxiliary connection device in the system and the connection relationship with other cell treatment devices can be adaptively adjusted according to the cell treatment processes. For example, the first cell treatment device 610 may be directly connected to the second cell treatment device 630, so that the first auxiliary connection device 620 is omitted. As another example, an input end of the first cell treatment device 610 (the left side of the first cell treatment device 610 in FIG. 6) may also be connected to the auxiliary connection device. As another example, the auxiliary connection device connected to an output end of the first cell treatment device 610 is not limited to the first auxiliary connection device 620 shown in FIG. 6, but may also be connected to other auxiliary connection devices, and the first auxiliary connection device 620 may be connected in parallel or in series with the other auxiliary connection devices. The specific structures of the first auxiliary connection device 620 and the second auxiliary connection device 640 can be referred to the foregoing with respect to the auxiliary connection device 100. In some embodiments, the first auxiliary connection device 620 and the second auxiliary connection device 640 may be of the same structure. In some embodiments, the first auxiliary connection device 620 and the second auxiliary connection device 640 may be of different structures. For example, the first auxiliary connection device 620 may include a power unit, a liquid transfer tubing assembly, a sampling unit, and a thermal insulation unit; and the second auxiliary connection device 640 may include only a power unit and a liquid transfer tubing assembly. It should be noted that the structure of the auxiliary connection device and its components can be adaptively adjusted according to the cell treatment processes. The first cell treatment device 610, the second cell treatment device 630, and the third cell treatment device 650 may be the same cell treatment device or different cell treatment devices. The auxiliary connection devices (e.g., the first auxiliary connection device 620 and the second auxiliary connection device 640) may flexibly combine the cell treatment devices according to different cell treatment processes, thereby forming combinations suitable for various application scenarios. For example, when the cell treatment system is used for pre-treatment of cells, the first cell treatment device 610 may be a cell washing device, the second cell treatment device 630 may be a cell incubation device, and the third cell treatment device 650 may be a cell sorting device. The auxiliary connection device may flexibly connect the different cell treatment devices described above, so as to complete the washing, incubation, and sorting processing of cells.

Possible beneficial effects of the auxiliary connection device disclosed in the present application include, but are not limited to: (1) the auxiliary connection device realizes the transfer of the solution through the first liquid transfer tubing in the liquid transfer tubing assembly; and in the solution transfer process, the auxiliary connection device can provide a closed and integrated confined space for the solution, thus reducing the contact between the solution and the external environment, decreasing the risk of the solution being contaminated, and increasing the success rate of the cell experiments or products; and (2) with the auxiliary connection device, the number of transfers of the solution in the external environment is decreased while workload of the staff is reduced greatly, and efficiency of the cell experiments or production is improved.

The basic concepts have been described above, and it will be apparent to those skilled in the art that the foregoing detailed disclosure is intended to be exemplary only and does not constitute a limitation to this specification. Although not explicitly stated herein, those skilled in the art may make various modifications, improvements and amendments to this specification. Such modifications, improvements and amendments are suggested in this specification, and thus remain within the spirit and scope of the exemplary embodiments of this specification.

Meanwhile, this specification uses specific words to describe the embodiments of this specification. For example, "one embodiment", "an embodiment" and/or "some embodiments" mean a particular feature, structure or characteristic related to at least one embodiment of this specification. Hence, it should be emphasized and noted that "an embodiment" or "one embodiment" or "one alternative embodiment" mentioned two or more times in different positions in this specification does not necessarily refer to the same embodiment. Furthermore, some features, structures or characteristics in one or more embodiments of this specification may be appropriately combined.

Furthermore, unless explicitly stated in the claims, the order of processing elements and sequences, the use of numbers and letters, or the use of other names described in this specification is not intended to limit the order of the process or method of this specification. Although some currently considered useful embodiments of the invention have been discussed in the above disclosure by way of various examples, it should be understood that such details are only for illustrative purposes, and that the appended claims are not limited to the disclosed embodiments; rather, the claims are intended to cover all amendments and equivalent combinations that are in line with the spirit and scope of the embodiments of this specification. For example, although the system components described above can be implemented by means of hardware devices, they may also be implemented only by software solutions, such as installing the described system on an existing server or mobile device.

Similarly, it should be noted that in order to simplify the presentation of the disclosure of this specification, and thereby help to understand one or more embodiments of the invention, in the previous descriptions of the embodiments of this specification, various features are sometimes combined into one embodiment, the accompanying drawings, or descriptions thereof. However, this method of disclosure does not imply that the subject of this specification requires more features than those mentioned in the claims. In fact, the embodiments have fewer features than all features of an individual embodiment disclosed above.

In some embodiments, numbers for describing components, properties and quantities are used. It should be understood that such numbers for describing the embodiments are modified by the modifiers "about", "approximately" or "substantially" in some examples. Unless otherwise stated, the modifiers "about", "approximately" or "substantially" indicate that the number is allowed to vary by ± 20%. Accordingly, in some embodiments, numerical parameters used in the specification and claims are approximate values that may vary depending on desired features of individual embodiments. In some embodiments, with regard to the numerical parameters, the specified number of significant digits should be taken into account, and a general digit retaining method should be used. Although numerical ranges and parameters for determining the breadth of ranges in some embodiments of this specification are approximate values, such numerical values should be set as accurate as possible in feasible ranges in the specific embodiments.

Each patent, each patent application, each patent application publication and other materials, such as articles, books, instructions, publications, documents, etc. cited in this specification are hereby incorporated by reference into this specification in its entirety. Application history documents that are inconsistent with or conflict with the contents of this specification are excluded, and documents (currently or later appended to this specification) that limit the broadest scope of the claims of this specification are also excluded. It should be noted that if there is any inconsistency or conflict between the descriptions, definitions, and/or the use of terms in the accompanying materials of this specification and the contents of this specification, the descriptions, definitions, and/or the use of terms in this specification shall prevail.

Finally, it should be understood that the embodiments described in this specification are only intended to illustrate the principles of the embodiments of this specification. Other variations may also fall within the scope of this specification. Thus, by way of example but not limitation, alternative configurations of the embodiments of this specification can be considered consistent with the teachings of this specification. Accordingly, the embodiments of this specification are not limited to those explicitly presented and described in this specification.

## Claims

1. An auxiliary connection device, **characterized by** comprising: a power unit and a liquid transfer tubing assembly, wherein the liquid transfer tubing assembly comprises a first liquid transfer tubing, the first liquid transfer tubing comprises a first port and a second port, the power unit engages with the first liquid transfer tubing, and the power unit is located at the first liquid transfer tubing between the first port and the second port.

2. The auxiliary connection device according to claim 1, **characterized in that** the device comprises a temporary storage unit connected to the liquid transfer tubing assembly, the liquid transfer tubing assembly comprises a second liquid transfer tubing, and the second liquid transfer tubing is connected to the temporary storage unit and the first liquid transfer tubing; a joint of the first liquid transfer tubing and the second liquid transfer tubing is located between the second port and the power unit; and
the liquid transfer tubing assembly comprises a third liquid transfer tubing, the third liquid transfer tubing is connected to the second liquid transfer tubing and the first liquid transfer tubing, and a joint of the third liquid transfer tubing and the first liquid transfer tubing is located between the first port and the power unit.

3. The auxiliary connection device according to claim 2, **characterized in that** the first liquid transfer tubing between the first port and the power unit is provided with a first valve, the first liquid transfer tubing between the second port and the power unit is provided with a second valve, the second liquid transfer tubing is provided with a third valve, and the third liquid transfer tubing is provided with a fourth valve.

4. The auxiliary connection device according to claim 3, **characterized in that** the device comprises a casing, and the first liquid transfer tubing, the second liquid transfer tubing, and the third liquid transfer tubing are distributed on a side wall of the casing.

5. The auxiliary connection device according to claim 4, **characterized in that** the first valve, the second valve, the third valve and the fourth valve are pinch valves, the pinch valves are connected to the side wall of the casing, and the pinch valves are located on an outer side of the liquid transfer tubing assembly.

6. The auxiliary connection device according to claim 5, **characterized in that** the power unit is a peristaltic pump, the peristaltic pump is located on the casing, and the first liquid transfer tubing engages with a pump head of the peristaltic pump.

7. The auxiliary connection device according to claim 5, **characterized in that** the device further comprises a thermal insulation unit for providing a preset temperature, the thermal insulation unit is located in the casing, and the temporary storage unit is located in the thermal insulation unit.

8. The auxiliary connection device according to claim 7, **characterized in that** the device further comprises a sampling unit, the liquid transfer tubing assembly comprises a fourth liquid transfer tubing, the fourth liquid transfer tubing is connected to the sampling unit and the first liquid transfer tubing, and a joint of the fourth liquid transfer tubing and the first liquid transfer tubing is located between the second port and the power unit.

9. The auxiliary connection device according to claim 8, **characterized in that** the fourth liquid transfer tubing is provided with a fifth valve that controls the on/off state of the fourth liquid transfer tubing, and the fifth valve is a pinch valve, is connected to the casing, and is located on an outer side of the fourth liquid transfer tubing.

10. The auxiliary connection device according to claim 9, **characterized in that** the sampling unit is located in the thermal insulation unit.

11. The auxiliary connection device according to claim 8, **characterized in that** the device further comprises a heating assembly, the heating assembly is located on the side wall of the casing, and the heating assembly is configured to cut off the fourth liquid transfer tubing and seal the cut end of the fourth liquid transfer tubing.

12. The auxiliary connection device according to claim 11, **characterized in that** the heating assembly is a heat sealing machine.

13. The auxiliary connection device according to claim 1, **characterized in that** the auxiliary connection device comprises a detection unit, the detection unit is located on an outer side of the first liquid transfer tubing between the first port and the power unit, and the detection unit is configured to determine whether there is a solution in the first liquid transfer tubing between the first port and the power unit.

14. The auxiliary connection device according to claim 13, **characterized in that** the detection unit is a bubble sensor, the bubble sensor is located on the outer side of the first liquid transfer tubing, the bubble sensor determines, based on ultrasound, whether there is a solution in the first liquid transfer tubing between the first port and the power unit.

15. The auxiliary connection device according to claim 14, **characterized in that** the device further comprises an information prompting unit connected to the detection unit, and the information prompting unit sends a prompt message in response to a feedback from the detection unit.

16. The auxiliary connection device according to claim 2, **characterized in that** the device further comprises a rinsing unit, the liquid transfer tubing assembly comprises a fifth liquid transfer tubing, one end of the fifth liquid transfer tubing is connected to the rinsing unit, and the other end of the fifth liquid transfer tubing is connected to the third liquid transfer tubing or the second liquid transfer tubing.

17. The auxiliary connection device according to claim 16, **characterized in that** the fifth liquid transfer tubing is provided with a sixth valve.

18. The auxiliary connection device according to claim 16, **characterized in that** the second liquid transfer tubing comprises a first sub-tubing and a second sub-tubing connected in sequence, a port of the first sub-tubing is connected to the temporary storage unit, a port of the second sub-tubing is connected to the first liquid transfer tubing, the third valve is located on the first sub-tubing, and the second sub-tubing is provided with a seventh valve.

19. The auxiliary connection device according to claim 16, **characterized in that** the device further comprises a supporting member, and the rinsing unit is disposed on the supporting member.
